# EUROPEAN PATENT SPECIFICATION

(11) **EP 0 679 165 B1**
(45) Date of publication and mention of the grant of the patent: **18.04.2001**
(21) Application number: 94907824.0
(22) Date of filing: 18.01.1994
(51) Int. Cl.: C08G 59/24, C07D 303/24

(54) **SUBSTITUTED RESORCINOL-BASED EPOXY RESINS**
EPOXYDHARZE AUF DER BASIS VON SUBSTIUIERTEM RESORCINOL
RESINES EPOXY A BASE DE RESORCINOL SUBSTITUE

(30) Priority: 15.01.1993 US 3987
(43) Date of publication of application: 02.11.1995
(73) Proprietor: INDSPEC CHEMICAL CORPORATION, Pittsburgh, PA 15238 (US)
(72) Inventor: DURAIRAJ, Raj, B., Monroeville, PA 15146 (US); TACKIE, Michael, N., Murraysville, PA 15668 (US)
(74) Representative: Bond, Bentley George
(86) International application number: PCT/US94/00556
(87) International publication number: WO 94/15986

(56) References cited:
- EP-A- 0 477 666
- FR-A- 2 496 675
- US-A- 2 739 160
- US-A- 2 892 849
- US-A- 4 842 910
- DATABASE WPI Derwent Publications Ltd., London, GB; AN 92-188631 & JP,A,4 122 715 (NIPPON KAXAKU KK) 23 April 1992
- DATABASE WPI Derwent Publications Ltd., London, GB; AN 86-235780 & JP,A,61 165 379 (SANYO KOKUSAKU PULP) 26 July 1986
- CHEMICAL ABSTRACTS, vol. 86, no. 22, 30 May 1977, Columbus, Ohio, US; abstract no. 156446, TAKAGI, Y E.A. 'Epoxides' page 35 ; & JP,A,51 143 633 (CEMEDINE CO., LTD) 10 December 1976
- DATABASE WPI Derwent Publications Ltd., London, GB; AN 86-065425 & JP,A,61 016 919 (TOTO KASEI KK) 24 January 1986
- DATABASE WPI Derwent Publications Ltd., London, GB; AN 68-30251Q & SU,A,215 366 (KH KH NURSKE)
- Handbook of Epoxy Resins, Lee and Neville, 1982, p.4-13,4-14.

## Description

### 1. Field of the Invention

This invention relates to substituted resorcinol-based epoxy resins having improved physical and mechanical properties such as a reduced ability to absorb moisture, reduced toxicity, high glass transition temperature, good processability and less volatility.

### 2. Brief Description of the Prior Art

Epoxy resins are a class of thermoset resins with a wide range of applications. For example, epoxy resins are employed in composite fabrication in both wet and dry lay-up techniques well known to those skilled in the art for the electrical, aerospace, filament winding and hardware industries. The disadvantage of many epoxy resins is their ability to absorb high amounts of moisture when they are used in both wet and dry composite fabrication. Obtaining an epoxy resin having a low moisture absorption characteristic, however, in many cases, has led to a compromise in other desirable physical and mechanical properties such as, for example, high glass transition temperature, ease of processability, flexural properties, tensile properties and curing conditions.

Epoxy resins are also used extensively in adhesive formulations and matrix resins for advanced composites. Demanding applications such as modern advanced composites fabrications require materials with low viscosity for easier processing, relatively faster cure for minimizing processing costs in the fabrication of industrial goods and low toxicity to reduce worker risks from exposure during manufacturing operations. Once fabricated, it is necessary for the parts made using epoxy resins to exhibit good physical properties such as high glass transition temperature (Tg) and mechanical properties like flexural, tensile and fracture toughness properties to maximize parts life and minimize in-service failure.

Although the epoxy resins provide the best attainable mechanical properties with carbon-fiber reinforced composite systems, higher levels of moisture absorption of these epoxy resins under hot/wet conditions deteriorate their properties. Therefore, epoxy resin producers are constantly developing new epoxy resins that can enhance physical and mechanical properties of both cured and uncured systems.

Resorcinol diglycidyl ether (RDGE, unsubstituted) resin offers the unique advantages of having very low viscosity and high reactivity towards various curing agents. This low viscosity unsubstituted-resorcinol epoxy shows excellent cured physical and mechanical properties in advanced composites. But, unfortunately, this low viscosity resorcinol epoxy has been reported to cause skin cancer. The study of Kotin et al. (published in Cancer, vol. 9, p. 910, 1956 and Proceedings of the American Association for Cancer Research, Vol. 2, p. 229, 1957) on the carcinogenicity of 20 epoxy resins indicated that the unsubstituted resorcinol epoxy resin (RDGE) produced skin tumors in rats and mice.

It is well known that all epoxy resins contain the epoxide, oxirane or ethoxylene group: 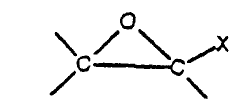 wherein X represents the point of attachment to the remainder of the resin molecule. The epoxide functionality generally appears in the form: 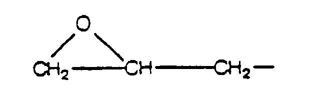 known by those skilled in the art as the glycidyl group which is attached to the remainder of a compound by such as, for example, an oxygen (i.e., glycidyl ether), nitrogen (i.e., glycidyl amine), or carboxyl group (i.e., glycidyl ester).

U.S. Patent No. 2,892,849 discloses a process for preparing epoxyalkyl aryl ethers, including resorcinol diglycidyl ether (RDGE).

U.S. Patent No. 4,916,202 discloses an epoxy resin having glycidyl amine groups. This patent discloses epoxy resins made from aliphatic amines, sulfonamides, and aromatic amines.

Japanese Patent No. -026173 discloses a process for making an epoxy resin from a carboxylic acid derivative of resorcinol such as Beta-resorcylic acid.

Lee and Neville in Handbook of Epoxy Resins, McGraw-Hill, 1967 and May and Tanaka in Epoxy Resins - Chemistry and Technology, Marcel-Dekker, 1973, have disclosed various kinds of epoxy resins, cured and uncured physical and mechanical properties with various curing agents and also products obtained from these resins.

Japanese Patent No. -020172 discloses the process of making an epoxy resin from the reaction of another resorcinol derivative namely 4,4'-thiodiresorcinol with epichlorohydrin.

U.S. Patent No. 2,467,171 discloses stereoisomeric 1,3-diglycidyloxybenzenes (RDGE) which have been known for many years.

Industrial and Engineering Chemistry, Volume 52, No. 4, April 1960, pp. 324-325 discloses epoxy resins from resorcinol-acetone condensation products.

U.S. Patent No. 3,291,837 discloses the preparation of monoepoxy compounds from benzoyl resorcinol and their application towards U.V. stabilizers.

U.S. Patent No. 4,656,207 discloses resorcinol-based epoxy compounds but not benzoyl resorcinol diglycidyl ether compounds.

WPI abstract AN92-188631 (JP-A-4122715) describes conductor sealing compositions comprising an epoxy resin component derived from substituted resorcinols.

WPI abstract AN86-235780 (JP-A-61165379) describes 4,6-dibromo-2-methyl-1,3-phenylene diglycidyl ether useful as an insulating material.

FR-A-2496675 describes (Claims 1,2) polyether resins including those derived from substituted resorcinols.

Chemical Abstracts, Vol. 86, No. 156446m describes epoxy resins derived from the diglycidyl ethers of 4-methyl- or 4-ethyl-resorcinol.

WPI abstract AN-68-30215 (SU-A-215366) describes coating, potting or insulating compositions comprising diglycidyl ethers of an alkyl resorcinol mixture which includes 5-methyl resorcinol and, for example, allyl resorcinols. These compositions are said to have low toxicity.

In spite of these prior art disclosures, there remains a very real and substantial need for a resorcinol-based epoxy resin having lower toxicity, enhanced curing properties with various types of curing agents, good mechanical properties like flexural, tensile, and fracture toughness and physical properties like low moisture absorption, high glass transition temperature and low volatility.

According to the present invention, there is provided a curable substituted resorcinol-based epoxy resin having the structural formula: 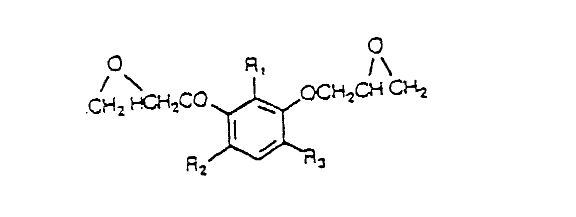 wherein at least one of R₁, R₂ and R₃ is selected from (a) aralkyl groups, (b) benzoyl groups and (c) substituted benzoyl groups having the structure: 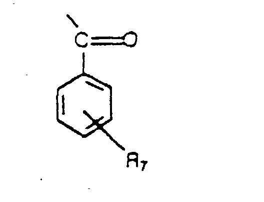 wherein R₇ is selected from the group consisting of halogen and an alkyl group having 1 to 4 carbon atoms.

The aralkyl group can have the following structure: 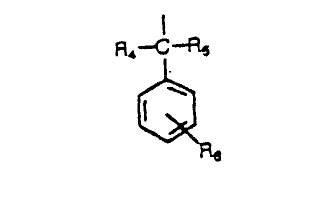 wherein each of R₄, R₅ and R₆, which are the same or different, is selected from hydrogen, an alkyl group having 1 to 4 carbon atoms, and halogen.

Preferably R₄ is methyl, R₅ is either hydrogen or methyl and R₆ is hydrogen.

The present invention provides new substituted-resorcinol based epoxy resins having reduced toxicity and reduced moisture absorption character while maintaining excellent cured physical and mechanical properties like the unsubstituted resorcinol epoxy.

The present invention also provides curable, substituted resorcinol-based epoxy resins having reduced toxicity, improved physical and mechanical properties including a substantially high glass transition temperature, a reduced moisture absorption capability, enhanced flexural, tensile properties with thermoplastic tougheners, and fracture toughness, and a substantially low viscosity.

The present invention provides a resorcinol-based epoxy resin that may be cured with conventional curing agents.

The present invention makes it possible, in view of the present environmental concerns of society, to make less volatile substituted resorcinol-based epoxy resin compounds that are characterized as less toxic compared to unsubstituted-resorcinol epoxy resins.

The present invention provides less toxic substituted resorcinol-based epoxy resins having physical and mechanical properties equal to or better than unsubstituted resorcinol epoxy resins, which may be potential alternatives in applications such as adhesive formulations and matrix resins for advanced composites.

The present invention provides less toxic substituted resorcinol-based epoxy resins capable of enhancing the fracture toughness of a cured epoxy matrix, whilst maintaining the high flexural and tensile modulus like relatively highly toxic unsubstituted resorcinol epoxy resin.

The present invention provides less toxic substituted resorcinol based epoxy resins that may be employed in the applications of coatings, laminates, composites, adhesives, castings, molded and encapsulated articles.

The present invention is directed to improvements in the physical and mechanical properties such as, for example, the low moisture absorption capability, high glass transition temperature, tensile, flexural and fracture toughness properties, low volatility, high reactivity and low enough viscosity to reduce toxicity of the selected curable substituted-resorcinol based epoxy resins.

As used herein, in the flexural and tensile data, σ is a measure of strength, E is the modulus and ε is elongation. K_{IC} is the fracture strength or toughness and G_{IC} is the fracture energy. Tg is the glass transition temperature or softening temperature of the substituted epoxy resin of this invention.

The epoxy resins of the present invention can be cured in a conventional manner. Suitable hardeners for the epoxy resins of the present invention include dicyandiamide, aromatic amines such as diamino diphenylsulfone (hereinafter "DDS") which has the formula: 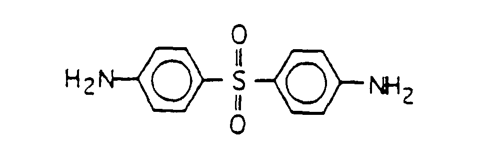 and 4,4'-[1,4-phenylene(1-methylethylidene)]-bis(2,6-dimethyl benzenamine) (Shell Development Company's curing agent 1062-M; hereinafter 1062-M) which has the following formula: 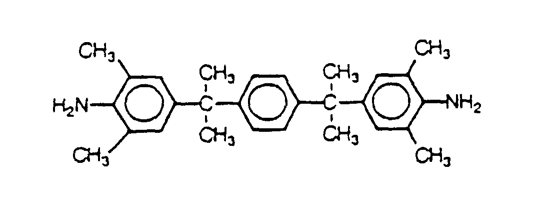 and polycarboxylic acid anhydrides. The preferred curing agents are DDS and 1062-M.

The amount of curing agent employed to cure the epoxy resins of the present invention will approximate the quantities employed with the presently used commercial resins such as EPON 828, EPON HPT 1071, EPON HPT 1072, EPON HPT 1079 and MY-720. Depending on the nature of the curing agent, curing can be performed at room temperature or at elevated temperatures. Curing provides a cross-linked polymer network which is infusible and intractable.

It will be appreciated by those skilled in the art that cures normally involve an added accelerator such as, for example, benzyldimethylamine (BDMA) and boron trifluoride in the form of its monoethylamine, piperidine or methylimidazole complex. The epoxy resins of the present invention may also be mixed with conventional epoxy resins prior to curing.

The epoxy resins of this invention can be toughened with functionally terminated acrylonitrile-butadiene copolymers, amine terminated propylene oxide oligomers, functionally terminated siloxane oligomers, and amine terminated thermoplastic tougheners such as polyethersulfone polymers. When amine terminated polyethersulfone was used as a toughener, the cured material of the present invention showed considerable improvements in fracture toughness while retaining a high modulus and high glass transition temperature (Tg). Suitable amine terminated polyethersulfones, that may be employed to enhance the toughness of the modified epoxy networks of this invention, preparations are disclosed in U.S. Patent No. 3,839,287 to Brode et al. and in McGrath et al., Polymer, Vol. 30, p. 1552 (1989).

It will be appreciated by those skilled in the art that while each commercially available epoxy resin has a number of advantageous properties, each resin, however, has a shortcoming in that it substantially lacks at least one of the hereinbefore mentioned properties generally required by those skilled in the art. By contrast, the epoxy resins of this invention generally have high glass transition temperatures, low water absorption properties, improved fracture toughness with amine terminated thermoplastic tougheners, less toxicity and improved flexural and tensile properties in comparison to the epoxy resin systems currently available. In this regard, the epoxy resins of this invention are well suited for most applications in which present commercial epoxy resins are employed including surface coatings and the manufacture of composites for aircraft industry. Laminates made from the epoxy resin matrices of this invention include the use of fibers such as, for example, glass, polyaramide and carbon. It will be understood by those skilled in the art that the epoxy resin composites of this invention may be fabricated using conventional wet lay-up and dry lay-up methods. The reinforced fiber may be impregnated with the epoxy resin matrix to produce a prepreg sheet. The epoxy resins of this invention are also suitable, for example, in the liquid form for filament winding, reaction injection molding, resin transfer molding and pultrusion.

The epoxy resins of the present invention may be excellent adhesives, particularly for bonding metals to metals, composites to composites and metal to composites when formulated properly using methods which are well known to those skilled in the art.

The epoxy resins of this invention are less volatile because of their bulky substituents and, therefore, less toxic as compared with other known unsubstituted resorcinol epoxy (RDGE) resins.

The present invention is more fully illustrated by the following non-limiting examples:

### EXAMPLE 1

This example illustrates the preparation of the epoxy resin of this invention having the structure shown in formula (I).

### Synthesis of Epoxy Resin from Benzoylresorcinol [RDGE-II]

Benzoylresorcinol (240.8 grams; 1.125 moles) and epichlorohydrin (1041 grams; 11.25 moles) were placed in a 3-liter round bottomed flask fitted with a stirrer, thermometer, Dean Stark condenser and an addition funnel. The contents of the flask were heated to about 100°-125° Centigrade (C) and an aqueous sodium hydroxide solution (50% W/W; 183 grams; 2.29 moles) was slowly added into the refluxing solution of benzoylresorcinol and the epichlorohydrin. While continuously removing the azeotropic water, the separated epichlorohydrin was continuously returned to the reaction flask. The addition time of sodium hydroxide was between about 1 to 1-1/2 hours and the time required for the complete removal of water (132 grams) was about 2 to 2-1/2 hours. After this, the excess epichlorohydrin was distilled out first under atmospheric pressure and then under vacuum distillation conditions [maximum pot temperature about 130°-135°C and vacuum about 6.5-9.9 KPa (27-28 inches (") of mercury (Hg))]. Then about 1200 ml of acetone was added to the reaction flask and the contents were refluxed for about 15 minutes to dissolve the epoxide. Finally, the salt, sodium chloride was filtered and the solvent acetone was distilled out using both atmospheric and vacuum distillation conditions (temperature about 95°-97°C and vacuum about 6.5-9.9 KPa (27-28" of Hg)) to obtain 352 grams (yield = 96%) of epoxide having an epoxide equivalent weight (EEW) of 180.

### EXAMPLE 2

This example illustrates the epoxy resin of this invention having the structural formula (I).

### Synthesis of Epoxy Resin from Benzoylresorcinol [RDGE-II]

Benzoylresorcinol (272.9 grams; 1.275 moles) and epichlorohydrin (1180 grams; 12.75 moles) were placed in a 3-liter round bottomed flask fitted with a stirrer, thermometer, Dean Stark condenser and an additional funnel. The contents of the flask were heated to about 100°-125°C and an aqueous sodium hydroxide solution (50% W/W; 207.4 grams; 2.59 moles) was slowly added into the refluxing solution of benzoylresorcinol and epichlorohydrin. While continuously removing the azeotropic water, the separated epichlorohydrin was continuously returned to the reaction flask. The addition time of sodium hydroxide was between about 1 to 1-1/2 hours and the time required for the complete removal of water (149.6 grams) was about 2 to 2-1/2 hours. After this, the excess epichlorohydrin was distilled out first under atmospheric pressure and then vacuum distillation conditions (maximum pot temperature about 130°-135°C and vacuum about 6.5-9.9 KPa (27-28" of Hg)). Then about 1300 ml of acetone was added to the reaction flask and the contents were refluxed for about 15 minutes to dissolve the epoxide. Finally, the salt, sodium chloride was filtered and the solvent acetone was distilled out using both atmospheric and vacuum distillation conditions (temperature about 95°-97°C and vacuum about 6.5-9.9 KPa (27-28" of Hg)) to obtain 412 grams (yield = 99%) of epoxide having an epoxide equivalent weight of 194.

### EXAMPLE 3

This example illustrates the preparation of the epoxy resin of this invention having the structural formula (I).

### Synthesis of Epoxy Resin of Benzoylresorcinol [RDGE-II]

Benzoylresorcinol (272.9 grams; 1.275 moles) and epichlorohydrin (1180 grams; 12.75 moles) were placed in a 3-liter round bottomed flask fitted with a stirrer, thermometer, Dean Stark condenser and an addition funnel. The contents of the flask were heated to about 100°-125°C and an aqueous sodium hydroxide solution (50% W/W; 214.5 grams; 2.68 moles) was slowly added into the refluxing solution of benzoylresorcinol and epichlorohydrin. While continuously removing the azeotropic water, the separated epichlorohydrin was continuously returned to the reaction flask. The addition time of sodium hydroxide was between about 1 to 1-1/2 hours and the time required for the complete removal of water (153.2 grams) was about 2 to 2-1/2 hours. After this, the excess epichlorohydrin was distilled out first under atmospheric pressure and then vacuum distillation conditions (maximum pot temperature about 130°-135°C and vacuum about 6.5-9.9 KPa (27-28" of Hg)). Then about 1300 ml of acetone was added to the reaction flask and the contents were refluxed for about 15 minutes to dissolve the epoxide. Finally, the salt, sodium chloride was filtered and the solvent acetone was distilled out using both atmospheric and vacuum distillation conditions (temperature about 95°-97°C and vacuum about 6.5-9.9 KPa (27-28" of Hg)) to obtain 402.5 grams (yield = 97%) of epoxide having an epoxide equivalent weight of 195.

### EXAMPLE 4

This example illustrates the epoxy resin of this invention having structural formula (I).

### Synthesis of Epoxy Resin from Styryl-substituted Resorcinol

### Preparation of Styrenated Resorcinol

Into a 1-liter reaction flask equipped with a stirrer, thermometer, reflux condenser and addition funnel, 165.0 grams of resorcinol were charged and heated to about 120°-130°C. para-Toluene sulfonic acid (1.0 gram) was then added at about 120°C and mixed for about 5 minutes. Then 234.5 grams of styrene (2.25 moles) were slowly added to the molten resorcinol dropwise over a period of about 1-1/2 hours. The temperature of the reaction mixture was maintained between about 115°-135°C during the styrene addition. After all of the styrene had been added, the reaction mixture was stirred at about 115°-135°C for about an additional 1 to 1-1/2 hours. At the end of this period 0.5 grams of 50% W/W sodium hydroxide solution was added to neutralize the acid before cooling to room temperature. Structural characterization using infrared spectroscopy (IR) and nuclear magnetic resonance spectroscopy (NMR) of the above reaction product indicated the presence of 1.5 styryl groups per resorcinol molecule. Analysis employing liquid chromatography (LC) and gas chromatography (GC) showed the presence of 1.4 weight percent unreacted resorcinol and also 0.01 weight percent unreacted styrene in the final product.

### Preparation of an Epoxy Resin from styrenated Resorcinol [DGESR]

Styryl resorcinol (359.0 grams; 1.35 moles) and epichlorohydrin (1249 grams; 13.5 moles) were placed in a 5-liter round bottomed flask fitted with a stirrer, thermometer, Dean Stark condenser and an additional funnel. The contents of the flask were heated to about 100°-125°C and an aqueous sodium hydroxide solution (50% W/W; 226.8 grams; 2.84 moles) was slowly added into the refluxing solution of styryl resorcinol and epichlorohydrin. While continuously removing the azeotropic water, the separated epichlorohydrin was continuously returned to the reaction flask. The addition time of sodium hydroxide was between about 1 to 1-1/2 hours and the time required for the complete removal of water (155 grams) was about 2 to 2-1/2 hours. After this, the excess epichlorohydrin was distilled out first under atmospheric pressure and then vacuum distillation conditions (maximum pot temperature about 130°-135°C and vacuum about 6.5-9.9 KPa (27-28" of Hg)). Then about 1500 ml of acetone was added to the reaction flask and the contents were refluxed for about 15 minutes to dissolve the epoxide. Finally, the salt, sodium chloride was filtered and the solvent acetone was distilled out using both atmospheric and vacuum distillation conditions (temperature about 95°-97°C and vacuum about 6.5-9.9 KPa (27-28" of Hg)) to obtain 514.5 grams of epoxide having an epoxide equivalent weight of 242. Structural characterization by IR/NMR analysis of the epoxide showed 1.83 epoxide groups per molecule.

### EXAMPLE 5

This example illustrates the epoxy resin of this invention having the structural formula (I).

### Synthesis of Epoxy Resin from Styryl-substituted Resorcinol [DGESR]

Into a 1-litre reaction kettle fitted with a stirrer, thermometer, Dean Stark reflux condenser and an addition funnel, 33.0 grams of resorcinol (0.3 moles) were placed. The kettle was then heated to melt the solid resorcinol. While maintaining the temperature of molten resorcinol between about 130° and 145°C, the catalyst p-toluenesulfonic acid (0.2 grams) was added first followed by the slow addition of 46.9 grams of styrene (0.45 moles) over a period of about 1/2 hour. After the styrene addition the reaction mixture was stirred at this temperature for about an additional 1.0 hour. Finally 0.2 grams of sodium hydroxide (50% W/W) was added to neutralize the acid catalyst. The styryl-substituted resorcinol made was cooled to about room temperature before adding 227.5 grams of epichlorohydrin (3.0 moles). The contents of the kettle were again heated to about 100°-125°C and an aqueous sodium hydroxide solution (50% W/W; 50.4 grams; 0.63 moles) was slowly added into the refluxing solution of styryl resorcinol and epichlorohydrin. While continuously removing the azeotropic water, the separated epichlorohydrin was continuously returned to the reaction flask. The addition time of sodium hydroxide was about 1 to 1-1/2 hours and the time required for the complete removal of water was about 2 to 2-1/2 hours. After this, the excess epichlorohydrin was distilled out first under atmospheric pressure and then vacuum distillation conditions (maximum pot temperature about 130°-135°C and vacuum about 6.5-9.9 KPa (27-28" of Hg)). Then, about 300 ml of acetone was added to the reaction flask and the contents were refluxed for about 15 minutes to dissolve the epoxide. Finally, the salt, sodium chloride was filtered and the solvent acetone was distilled out using both atmospheric and vacuum distillation conditions (temperature about 95°-97°C and vacuum about 6.5-9.9 KPa (27-28" of Hg)) to obtain 104.3 grams of epoxide (yield = 92%) having an epoxide equivalent weight of 215. Structural characterization by IR/NMR analysis of the epoxide showed 1.97 epoxide groups per molecule.

### EXAMPLE 6

This example illustrates the epoxy resin of this invention having the structural formula (I).

### Synthesis of Epoxy Resin from Alpha-Methylstyryl-substituted Resorcinol

### Preparation of Alpha-Methylstyryl Resorcinol

Into a 3-litre kettle fitted with a stirrer, thermometer, reflux condenser and an addition funnel 137.5 grams of resorcinol (1.25 moles), 10.0 grams of oxalic acid, 900 ml of heptane were placed. The contents of the flask were then heated to about 65°C. While maintaining the temperature of the resorcinol slurry between about 65° and 75°C, 312 grams of alpha-methyl styrene (2.6 moles) was added dropwise with stirring for about 2.0 hours. After the completion of the above addition, the temperature of the reaction mixture was raised to and maintained at about 95°C and for an additional period of about 2.0 hours while the reaction mixture was continuously stirred. The reaction mixture was cooled and the colorless product that separated was filtered, washed several times with distilled water and dried in air at about room temperature to obtain 376 grams of alpha-methylstyrylsubstituted resorcinol (yield = 84%). Structural characterization by IR/NMR analysis of the above reaction product showed the presence of 1.85 alpha-methylstyryl groups per resorcinol molecule.

### Preparation of an Epoxy resin from Alpha-Methylstyrenated Resorcinol [DGEMSR]

Alpha-methylstyryl resorcinol (65.6 grams; 0.2 moles) and epichlorohydrin (148 grams; 1.6 moles) were placed in a 500-ml round bottomed flask fitted with a stirrer, thermometer, Dean Stark condenser and an additional funnel. The contents of the flask were heated to about 100°-125°C and an aqueous sodium hydroxide solution (50% W/W; 36.8 grams; 0.46 moles) was slowly added into the refluxing solution of alpha-methylstyryl resorcinol and epichlorohydrin. While continuously removing the azeotropic water, the separated epichlorohydrin was continuously returned to the reaction flask. The addition time of sodium hydroxide was about 1.0 hour and the time required for the complete removal of water (25.6 grams) was about 1-1/2 to 2 hours. After this, the excess epichlorohydrin was distilled out first under atmospheric pressure and then vacuum distillation conditions (maximum pot temperature about 130°-135°C and vacuum about 6.5-9.9 KPa (27-28" of Hg)). Then about 200 ml of acetone was added to the reaction flask and the contents were refluxed for about 15 minutes to dissolve the epoxide. Finally, the salt, sodium chloride was filtered and the solvent acetone was distilled out using both atmospheric and vacuum distillation conditions (temperature about 95°-97°C and vacuum about 6.5-9.9 KPa (27-28" of Hg)) to obtain 88.0 grams of epoxide having an epoxide equivalent weight of 255. Structural characterization by IR/NMR analysis of the epoxide showed 1.94 epoxide groups per molecule.

### EXAMPLE 7

### Synthesis of Amine Terminated Poly (arylene ether sulfone) oligomers

Into a four necked round-bottomed flask fitted with a Dean-Stark trap, mechanical stirrer, thermometer and nitrogen inlet, bisphenol-A (11.41 grams; 0.05 mole), m-aminophenol (0.27 grams; 0.0025 mole), 4,4'-dichlorodiphenyl sulfone (14.72 grams; 0.051 mole), dried potassium carbonate (17.7 gram; 0.128 mole), N-methylpyrrolidone (160 ml.) and toluene (55 ml.) were added together. The reaction mixture was heated and taken to reflux and kept at this condition until all the water had been removed (approximately 4.0 hours). After this toluene was drawn from the Dean-Stark trap until the temperature increased to 155-160°C. The reaction was allowed to proceed for 8 hours, after which the solution was cooled and filtered to remove the inorganic salts, then coagulated in an excess of rapidly stirred methanol-water mixture. The precipitate, which separated out, was filtered and washed several times with water and then methanol to completely remove all the salt and solvent. The precipitate was then dried at 80°C under vacuum to obtain m-aminophenol terminated polyethersulfone oligomer as a solid product.

Structure of the resulting oligomers were confirmed by IR/NMR analysis. The number average molecular weight of the oligomers were determined by potentiometric titration of the endgroups. Differential Scanning Calorimetry (DSC) was used to determine the glass transition temperature (Tg) of the oligomers.

The two amine terminated polyethersulfone oligomers made from different molar ratios of bisphenol-A, dichlorodiphenyl sulfone and m-aminophenol (hereinafter Bisphenol-A Sulfone Toughener) had 3550 molecular weight (low; Tg = 125°C) and 13270 molecular weight (high; Tg = 162°C)

### EXAMPLE 8

Following Example 7, two amine terminated polyethersulfone oligomers were made using different molar ratios of resorcinol, dichlorodiphenyl sulfone and m-aminophenol reactants. These oligomers are hereinafter referred to as Resorcinol-Sulfone Toughener. The number average molecular weights of these two tougheners determined by titration are 3070 (low molecular weight) and 17860 (higher molecular weight) and had glass transition temperatures of 138°C (low) and 165°C (high).

### EXAMPLE 9

Following Example 7, two amine terminated tougheners were synthesized using 4-benzoyl resorcinol, dichlorodiphenyl sulfone and m-aminophenol reactants. These oligomer tougheners are hereinafter referred to as Benzoylresorcinol-sulfone Toughener. The number average molecular weights and Tg of these tougheners were found to be 5460 (low; Tg 109°C) and 12429 (high; Tg 112°C).

These amine terminated polyethersulfone oligomers are used as tougheners to enhance the fracture toughness properties of the epoxy resins of the present invention.

### EXAMPLE 10

The following Table 1 sets forth the physical properties of the epoxy resins of Examples 1 through 6. The viscosity was not measured for Examples 1 and 2. The viscosity for Example 3, however, determined at room temperature was found to be about 29 Pa^{•}S. It is important to note that this viscosity is higher than the unsubstituted-resorcinol epoxy resin (0.5 Pa^{•}S; RDGE from Shell Development company, Houston, Texas sold under the trade name of Heloxy 69). Therefore, the epoxy resin (RDGE-II) of the present invention is expected to be less toxic compared to RDGE due to lesser tendency to penetrate into the skin associated with higher viscosity.

Also, it is important to note that the commercial epoxy resins presently available for higher performance composites and other applications have relatively higher viscosities compared to the resins of the present invention. Therefore, the epoxy resin of this invention has a processing advantage over the present commercial epoxy resins. Because the resin of this invention has such a low viscosity, it will be understood by those skilled in the art that it is suitable for more automated manufacturing processes such as, for example, resin transfer molding, reaction injection molding and pultrusion.

**Table 1**

| Synthesis and Physical properties of Diglycidyl ether of Benzoyl resorcinol (RDGE-II) | | | |
|---|---|---|---|
| Example | 1 | 2 | 3 |
| Yield(%) | 96 | 99 | 97 |
| | | | |
| EEW | 180 | 194 | 195 |
| | | | |
| Total Chlorine (Wt. %) | 0.3 | 0.39 | 0.4 |
| | | | |
| Viscosity (Pa•s at 25°C) | - | - | 29 |
| | | | |
| LC/GC Analysis | | | |
| Acetone (Wt. %) | 0.3 | 0.2 | 0.7 |
| | | | |
| Epichlorohydrin (Wt. %) | 0.06 | 0.04 | 0.1 |

From the IR/NMR analysis it was found out that RDGE-II possess greater than 1.9 epoxy groups per molecule; thereby expected to show higher reactivity towards different curing agents.

### EXAMPLE 11

To study the neat resin mechanical properties of the substituted resorcinol epoxy resins of the present invention, the epoxy resins were blended with curing agents like diaminodiphenyl sulfone (DDS) and Shell's 1062-M curing agents at about 130-140°C and void-free castings were made from the molten resin blend using silicone molds.

The flexural specimens were measured 76.2 mm x 12.7 mm x 3.3 mm. Tensile dogbone specimens measured 152.4 mm x 12.7 mm x 3.3 mm with a 5.1 mm wide gage section. The mode I fracture toughness specimens measured 152.4 mm x 15.2 mm x 3.3 mm. Three 5 mm notches were cut into the specimens two inches apart, and a sharp razor blade was used to precrack them before fracture testing.

The standard epoxy resin cure cycle (cure 'a') was 2.0 hours at 150°C, followed by 4.0 hours at 200°C. Another cure cycle (cure 'b'), used solely for comparison of DDS and 1062-M in the unmodified resin, was 2.0 hours at 180°C followed by 2.0 hours at 210°C. These curing cycles are referred to as 'cure a' and 'cure b' respectively.

Summaries of the test results for the cured RDGE-II resins of this invention are shown in Table 2.

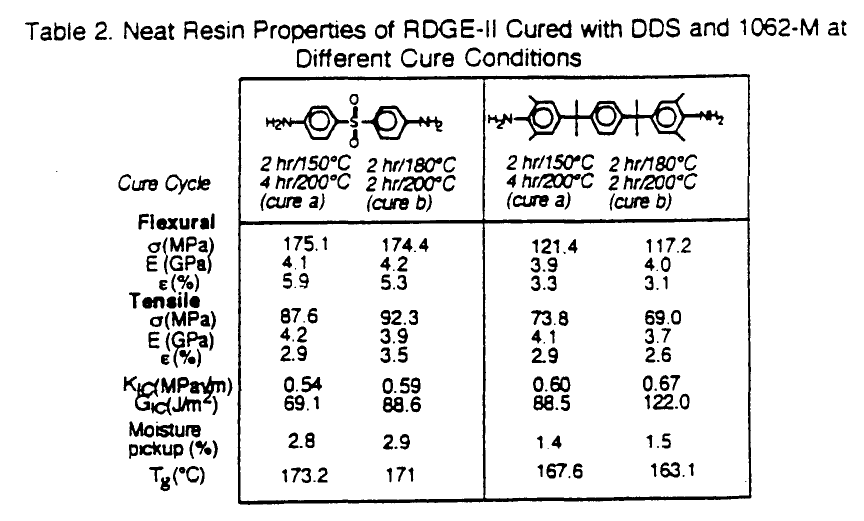

Mechanical properties like flexural and tensile properties are observed to be higher and the moisture pickup was found to be about 2.8 weight percent for DDS cured resins and about 1.4 weight percent for 1062-M cured resins after 48 hours of water boil. The fracture toughness (K_{IC} and G_{IC}) values indicate that these systems have to be toughened in order to use as matrix resins for tough composite parts fabrication.

### EXAMPLE 12

The mechanical properties of RDGE-II cured with DDS at different cure conditions are given in Table 3 hereinafter. High flexural strength and modulus are maintained irrespective of the cure conditions. In addition to mechanical properties, the Tg does not change significantly, indicating that this resin system is very forgiving of varying cure conditions.

**Table 3.**

| Neat Resin Properties of RDGE-II Cured with DDS at Different Cure Conditions | | | | |
|---|---|---|---|---|
| | RDGE-II : 100 g | | DDS: 35-36 g | |
| Cure cycle (h/°C) | 2/180 | 4/150 | 2/150 | 1/150 |
| Postcure | 2/210 | 7/200 | 4/200 | 3/225 |
| | | | | |

| Property **Flexural** | | | | |
|---|---|---|---|---|
| Strength (MPa) | 184.1 | 195.1 | 197.9 | 169.6 |
| Modulus (GPa) | 4.2 | 4.1 | 4.1 | 4.3 |
| Elongation (%) | 6.5 | 6.4 | 6.9 | 4.7 |
| | | | | |
| Glass Transition Temperature (T_{g} by TMA, °C) | 171.0 | 166.0 | 175.0 | 175.5 |
| | | | | |
| Moisture absorption (%, after 48 hours water boil) | 3.0 | ND | 3.1 | 3.0 |
| | | | | |
| Impact Strength | (0.5) | ND | (0.38) | (0.4) |
| (Notched Izod; (ft.-lb/in) | | | | |
| J/m | 26.69 J/m | | 20.28 J/m | 21.35 J/m |
| ND = Not Determined | | | | |

### EXAMPLE 13

The flexural measurements results on RDGE-II cured with DDS at elevated temperatures are given in Table 4 hereinbelow. As can be expected, both the flexural strength and modulus decreased as the temperature increased. At 121°C, 57% of the room temperature flexural strength and 50% of flexural modulus were retained, which is similar to the performance of other high performance epoxy resins.

**Table 4.**

| Elevated Temperature Mechanical Properties of RDGE-II Cured with DDS | | | | |
|---|---|---|---|---|
| Resin/Curing Agent: | RDGE: 100.0 g | DDS: 35-36 g | | |
| Cure Cycle (h/°C) | 2/150 | | | |
| Postcure | 4/200 | | | |
| | | | | |

| | | Temperature (°C) | | |
|---|---|---|---|---|
| | | 25 | 65.5 | 121.1 |
| **Flexural Property** | | | | |
| Strength (MPa) | | 186.4 | 158.6 | 106.9 |
| Modulus (GPa) | | 4.5 | 3.3 | 2.3 |
| Elongation (%) | | 4.6 | 6.9 | 5.9 |

### EXAMPLE 14

Flexural properties obtained after 48 hours water boil on the RDGE-II/DDS cured samples are presented in Table 5 hereinafter. From the results of the experiments, it was found that RDGE-II retains about 80% of its strength and 86-95% of its modulus even after water boil.

**Table 5.**

| Neat Resin Properties of RDGE-II Cured with DDS and after Water Boil* | | | | | | |
|---|---|---|---|---|---|---|
| Resin/Curing Agent: | RDGE-II: 100 g | | | DDS: 35-36 g | | |
| | | | | | | |
| Cure Cycle (h/°C) | | 2/180 | | | 2/150 | |
| Postcure | | 2/210 | | | 4/200 | |
| | | | | | | |

| **Flexural Property** | Dry | | Wet | Dry | | Wet |
|---|---|---|---|---|---|---|
| | | | | | | |
| Strength (MPa) | 173.7 | | 141.3 | 186.2 | | 144.8 |
| Modulus (GPa) | 4.4 | | 3.8 | 4.3 | | 4.1 |
| Elongation (%) | 5.4 | | 4.1 | 6.2 | | 4.3 |

| | | | | | | |
|---|---|---|---|---|---|---|
| *48 h water boil | | | | | | |

### EXAMPLE 15

The neat resin mechanical properties of RDGE-II and RDGE resins obtained are given in Table 6 hereinbelow. The curing agent used was DDS and both 'cure a' and 'cure b' conditions were used.

**Table 6.**

| Neat Resin Properties of RDGE and RDGE-II Cured with DDS | | | | |
|---|---|---|---|---|
| | RDGE-II | | RDGE | |
| Viscosity (Pa•s) | 2.9-3.0 at 50°C | | 0.5 at 25°C | |

| | *cure a* | *cure b* | *cure a* | *cure b* |
|---|---|---|---|---|
| **Flexural** | | | | |
| Strength (MPa) | 175.1 | 174.4 | 171.7 | 176.5 |
| E(GPa) | 4.1 | 4.2 | 4.0 | 3.9 |
| strain (%) | 5.9 | 5.3 | 7.4 | 6.6 |
| | | | | |

| **Tensile** | | | | |
|---|---|---|---|---|
| Strength (MPa) | 87.6 | 92.3 | 99.3 | 93.8 |
| E (GPa) | 4.2 | 3.9 | 3.9 | 4.3 |
| strain (%) | 2.9 | 3.5 | 5.1 | 4.9 |
| | | | | |
| **Moisture pickup (%)*** | 2.79 | 2.90 | 3.59 | 3.36 |
| **Tg** (°C) | 173.2 | 171.0 | 182.3 | 178.3 |

| | | | | |
|---|---|---|---|---|
| ^{*}After 48 h water boil | | | | |

From the results of the flexural and tensile data, it is very clear that the performance of RDGE-II is similar to RDGE. The Tg of RDGE-II is about 10°C lower than RDGE. This lower Tg value is due to the substitution on the benzene ring which results in a reduction in the crosslink density.

Another important result of the benzene ring substitution which leads to an improvement of RDGE-II over RDGE can be seen from the moisture pickup values. RDGE-II absorbs about 2.8% water compared to 3.6% for RDGE in the 48 hour water boil test. It is expected that this would translate into an improved elevated temperature performance of RDGE-II compared to RDGE.

### EXAMPLE 16

The following table (Table 7) shows the comparison of the mechanical properties of the present invention to that of the commercial resins.

**Table 7.**

| Neat Resin Mechanical properties Comparison Epoxy Resin = 100 g | | | | |
|---|---|---|---|---|
| Resin | RDGE-II | | ARALDITE XU MY 722 | DER 332 |
| Curing Agent (g) (DDS) | 35.0 | | 50.0 | 33.0 |
| | | | | |
| Cure Cycle, | 2/180 | 2/150 | 2/180 | 2/150 |
| Hours,/°C | 2/210 | 4/200 | 2/210 | 4/200 |
| | | | | |

| Flexural Properties (Dry) | | | | |
|---|---|---|---|---|
| Strength (MPa) | 173.7 | 186.2 | 125.7 | 125.7 |
| Modulus (GPa) | 4.4 | 4.3 | 4.1 | 3.0 |
| Strain (%) | 5.4 | 6.2 | - | 5.4 |
| | | | | |
| Moisture Absorption (%) (After 48 hours water boil) | 3.0 | - | 3.7 | - |

Table 7 shows the mechanical properties of cured Araldite XU MY 722 taken at 25°C and DER 332. Araldite XU MY 722 is commercially available from Ciba-Geigy corporation, Hawthorne, New York, U.S.A. and DER 332 is commercially available from Dow Chemical, U.S.A. Araldite was cured with aromatic diamine hardener HT 976 (DDS) commercially available from Ciba-Geigy Corporation. Table 7 shows that the cured resin of this invention (RDGE-II) and the cured Araldite resin and DER 332 were cured under a two-step cure cycle. From the results of the above table, it will be appreciated that the epoxy resin of this invention has improved flexural properties as compared to Araldite and DER 332. Further, another advantage of the epoxy resin of RDGE-II is that it has 3% moisture absorption after 48 hours of being boiled in water. In contrast, Araldite has a 3.7% moisture absorption.

### EXAMPLE 17

Neat resin castings of cured epoxy materials containing both an amine terminated polyarylether sulfone oligomers (Examples 7, 8 and 9) and curing agent were done as follows using high and low molecular weight tougheners. The toughener employed in the resin castings were 5, 10 and 15 weight percent of the final cured material. The epoxy resin was first mixed with the toughener at 120-135°C. The mixing times varied depending on the epoxy resin and on the toughener level. For example, when using RDGE-II and the bisphenol-A based toughener, complete mixing was accomplished after 2 hours at 120°C, while for RDGE, the mixing times could be as short as 15 minutes. After the toughener had formed a homogeneous solutions with the epoxy, a stoichiometric amount of DDS was added to the mixture and stirred at 130°c until it dissolved. The solution was then degassed under vacuum for 15 minutes. The RDGE-II resins with high loadings of toughener were vented to atmospheric pressure and degassed for several cycles in order to remove all the entrained air. After degassing, the resin was poured into preheated silicone molds and cured using 'cure a' conditions.

### EXAMPLE 18

Neat resin properties of RDGE-II and RDGE cured with DDS and amine terminated reactive bisphenol-A sulfone toughener are present in Tables 8 and 9 hereinbelow.

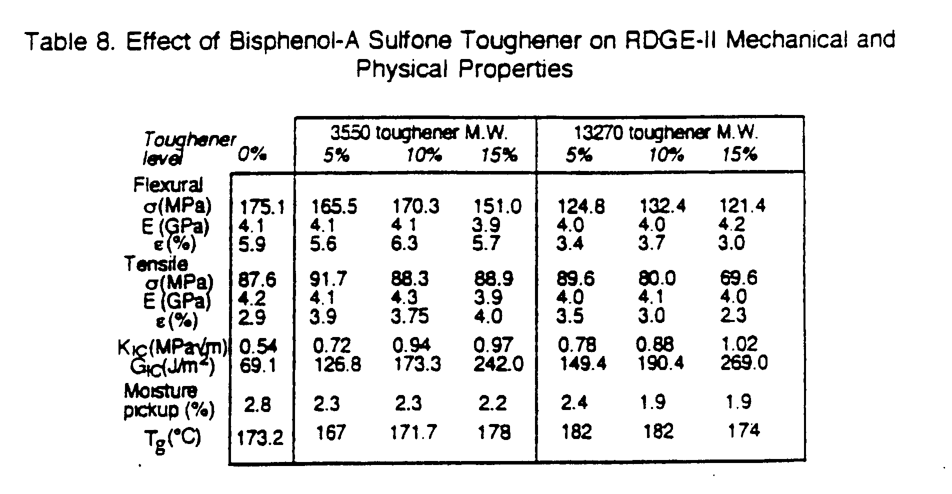

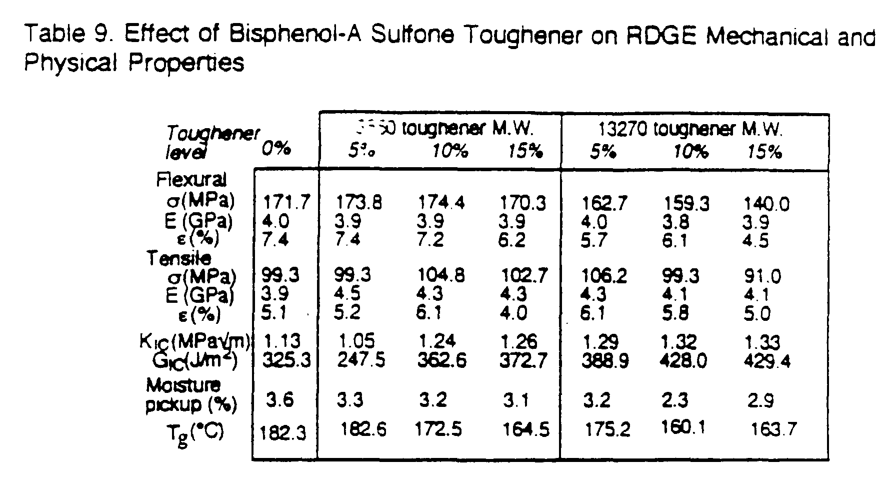

From the results of toughening experiments, the modulus of RDGE-II and RDGE remained essentially unchanged. This thermoplastic toughener had the most positive overall effect on RDGE-II compared to RDGE. In the case of RDGE-II, fracture energy increased from 69.1 J/m² in the unmodified resin to 269 J/m² with 15% of the higher molecular weight toughener. The fracture toughness increased almost 4 times but the Tg was maintained. With RDGE, the fracture toughness increased by 18% and Tg decreased by 20°C. These results indicated that the thermoplastic modified RDGE-II has a higher TG, higher modulus, lower moisture absorption (by almost 50%) and toughness properties comparable to RDGE.

### EXAMPLE 19

Neat resin properties of RDGE-II and RDGE cured with DDS and amine terminated reactive resorcinol sulfone toughener are presented in Tables 10 and 11 hereinbelow.

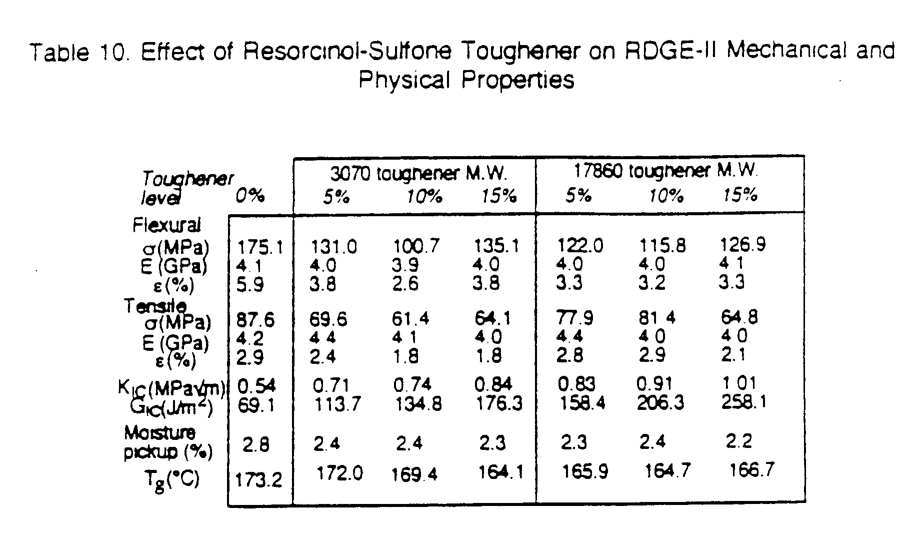

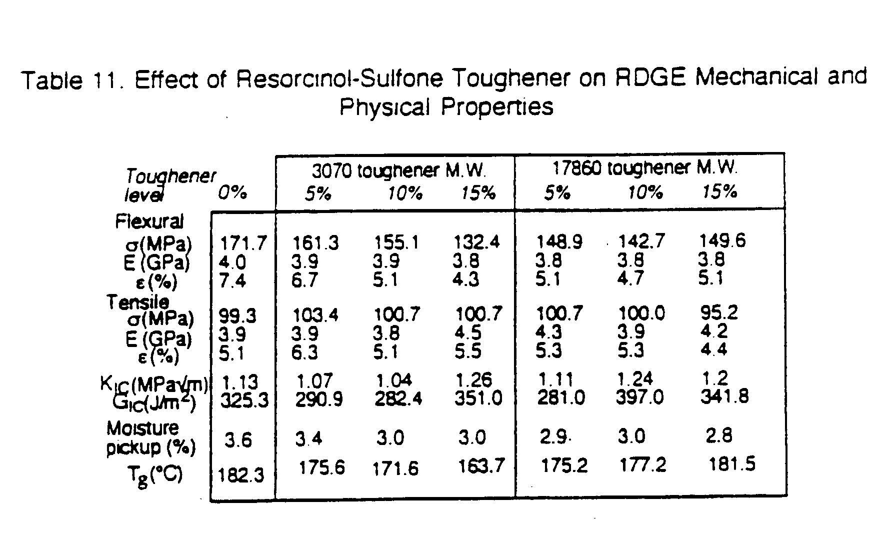

From the results of Tables 10 and 11, the resorcinol sulfone enhances the fracture energy of RDGE-II higher than that of RDGE where the toughener provides virtually no improvement. The resorcinol toughener also improves the lower moisture absorption characteristics of RDGE -II compared to RDGE resin.

### EXAMPLE 20

Neat resin properties of RDGE-II and RDGE cured with DDS and amine terminated reactive benzoyl resorcinol sulfone toughener are shown in Tables 12 and 13 hereinbelow.

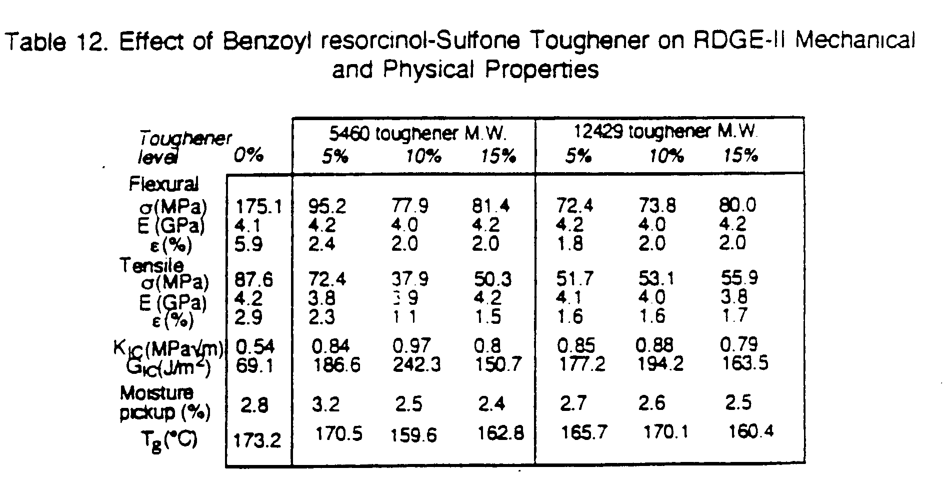

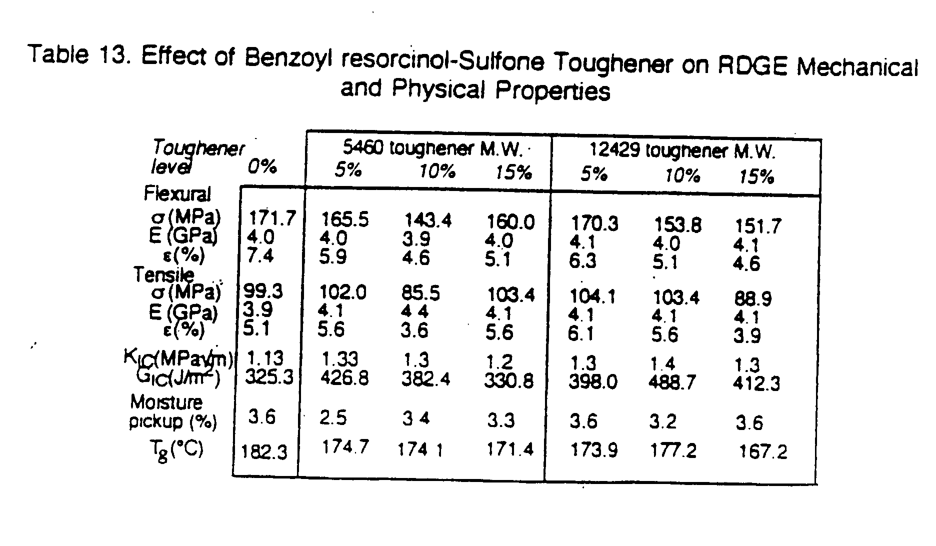

With benzoyl resorcinol sulfone toughener the flexural modulus of both RDGE-II and RDGE resins are maintained. Also, there is an improvement in fracture toughness and fracture energy in both RDGE-II and RDGE but the moisture pickup is unchanged from the neat resin without any added toughener.

### EXAMPLE 21

Table 14 shows the mechanical properties of diglycidylether of styrylresorcinol (DGESR) of Example 4 that has been cured with DDS. Table 14 hereinbelow shows that the epoxy resin had a very high flexural modulus of 4.7 mPa but had a low glass transition temperature of 128.8°C.

**Table 14.**

| Mechanical properties of DGESR Cured with DDS | |
|---|---|
| DGESR, g. (From Example 4) | 100 |
| DDS, g. | 27.5 |
| | |

| Flexural Properties (Dry) | |
|---|---|
| Strength (MPa) | 129.0 |
| Modulus (GPa) | 4.7 |
| Elongation (%) | 2.8 |
| | |

| Cure Cycle, Hours/°C | |
|---|---|
| (1) | 23/120 |
| (2) | 4/175 |
| | |

| Glass Transition | |
|---|---|
| Temperature, °C (TMA) Dry | 128.8 |

### EXAMPLE 22

Table 15 hereinbelow shows the mechanical properties of diglycidylether of alphamethylstyrylresorcinol (DGEMSR) of Example 6 that has been cured with DDS. Table 15 shows that the epoxy resin had an acceptable glass transition temperature of 164.8°C and had acceptable flexural properties. The epoxy resin had a 2% moisture absorption after 48 hours of water boil. It will be appreciated, therefore, that the epoxy resin has a very low moisture absorption ability. This property is particularly advantageous for a resin employed in an environment having a high humidity level.

**Table 15.**

| Mechanical Properties of DGEMSR Cured with DDS | |
|---|---|
| DGEMSR, g. (From Example 6) | 100 |
| DDS, g. | 20.5 |
| | |

| Flexural Properties (Dry) | |
|---|---|
| Strength (MPa) | 88.0 |
| Modulus (GPa) | 3.7 |
| Elongation (%) | 2.4 |
| | |

| Flexural Properties (Wet) (After 48-hours water boil) | |
|---|---|
| Strength (MPa) | 52.5 |
| Modulus (GPa) | 3.8 |
| Elongation (%) | 1.1 |
| | |

| Cure Cycle, Hours/°C | |
|---|---|
| (1) | 2/120 |
| (2) | 4/200 |
| | |

| Glass Transition | |
|---|---|
| Temperature, °C (TMA) Dry | 164.8 |
| | |

| Moisture Absorption (%) | |
|---|---|
| (After 48-hours water boil) | 2.0 |
| | |
| Impact Strength (Ft-lb/in) J/m (Notched Izod) | (0.48) 25.62 J/m |

### EXAMPLE 23

Table 16, below, shows the results of toxicity studies of both RDGE and RDGE-II as an eye irritant and dermal irritant on mice and rats.

**Table 16**

| | **RDGE** | **RDGE-II** |
|---|---|---|
| dermal irritant | (a) well-defined redness and swelling at one hour | (a) slight degree of redness and barely perceptible swelling at one hour |
| | | |
| | (b) no appreciable recovery over the 72-hour observation period | (b) complete recovery at 24 hours |
| | | |
| eye irritant | (a) a severe degree of irritation involving the cornea and deeper tissue | (a) light irritation to the conjunctiva but not the cornea or iris |
| | | |
| | (b) no recovery at 72 hours | (b) full recovery at 48 hours |

The data set forth hereinbefore and Table 17, hereinafter, which summarizes the results from the substituted resorcinol-based resins, clearly demonstrate that the epoxy resins of this invention have improved mechanical and physical properties over the presently commercially available epoxy resins.

**Table 17**

| **Summary of Results from Substituted Resorcinol Based Resins** | | | | |
|---|---|---|---|---|
| Epoxy Resin | RDGE | RDGE-II (Example 2) | DGESR (Example 5) | DGEMSR (Example 6) |
| Uncured Property | | | | |
| 1. Epoxy eq.weight | 130 | 180-195 | 215 | 255 |
| 2. Viscosity (Pa•s, 25°C) | 0.5 | 25.0-30.0 | 60.0 | N.D. |
| 3. Epoxy groups per molecule | >1.9 | 1.9-1.95 | 1.97 | 1.94 |
| | | | | |

| Cured Property | | | | |
|---|---|---|---|---|
| 1. Curing Agent Used* | DDS | DDS | DDS | DDS |
| | | | | |
| 2. Cure cycle (h/°C) | 2/150 | 2/150 | 23/120 | 2/120 |
| Postcure | 4/200 | 4/200 | 4/175 | 4/200 |
| | | | | |
| 3. Flexural | | | | |
| Strength (MPa) | 171.7 | 175.1 | 129.0 | 88.0 |
| Modulus(GPa) | 4.0 | 4.1 | 4.7 | 3.7 |
| Elongation (%) | 7.4 | 5.9 | 2.8 | 2.4 |
| | | | | |
| 4. T_{g}(°C) | 182.0 | 173.0 | 128.8 | 165.0 |
| | | | | |
| Moisture Absorption (%) after 48 hours water boil | 3.59 | 3.0 | N.D. | 2.0 |

| | | | | |
|---|---|---|---|---|
| * = Stoichiometric amount used. N.D. = Not determined. | | | | |

Whereas particular embodiments of this invention have been described above for purposes of illustration, it will be evident to those persons skilled in the art that numerous variations of the details of the present invention may be made without departing from the invention as defined in the appended claims.

## Claims

1. A curable substituted resorcinol-based epoxy resin having the structural formula: 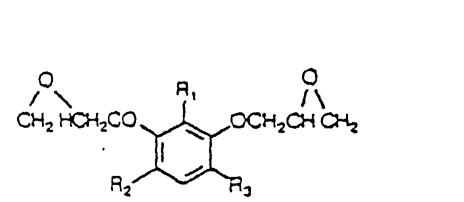 wherein at least one of R₁, R₂ and R₃ is selected from (a) aralkyl groups, (b) benzoyl groups and (c) substituted benzoyl groups having the structure: 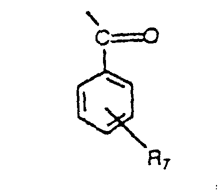 wherein R₇ is selected from the group consisting of halogen and an alkyl group having 1 to 4 carbon atoms.

2. A substituted resorcinol-based epoxy resin as claimed in claim 1, wherein the aralkyl group has the following structure: 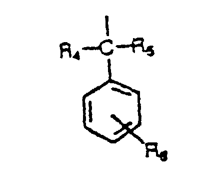 wherein at least one of R₄, R₅ and R₆ is selected from (a) hydrogen, (b) an alkyl group having 1 to 4 carbon atoms and (c) halogen.

3. A curable substituted resorcinol-based epoxy resin as claimed in claim 1, having a cougnener agent therein.

4. The use of a curable substituted resorcinol-based epoxy resin as claimed in Claim 3 as an adhesive to bond metal to metal, metal to composite, or composite to composite.

5. The use of a curable substituted resorcinol-based epoxy resin as claimed in Claim 3, in a coating formulation.

6. A curable substituted resorcinol-based epoxy resin as claimed in Claim 3, wherein the toughener agent is present in an amount of from 1% to 15% by weight.

7. A curable substituted resorcinol-based epoxy resin as claimed in Claim 2, wherein R₄ is methyl, R₅ is either hydrogen or methyl and R₆ is hydrogen.

8. A tough, durable composite part comprising a matrix resin, toughener and filler material; said matrix resin being comprised of a curable substituted resorcinol-based epoxy resin as claimed in claim 1.

## Patentansprüche

1. Härtbares, substituiertes Epoxyharz auf Resorcin-Basis mit der Strukturformel: 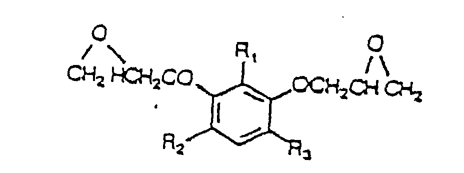 worin mindestens ein Rest aus R₁, R₂ und R₃ ausgewählt ist aus (a) Aralkylgruppen, (b) Benzoylgruppen und (c) substituierten Benzoylgruppen mit der Struktur: 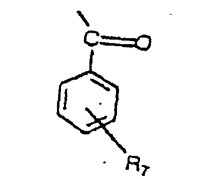 worin R₇ ausgewählt ist aus der Gruppe, bestehend aus Halogen und Alkylgruppen mit 1 bis 4 Kohlenstoffatomen.

2. Substituiertes Epoxyharz auf Resorcin-Basis nach Anspruch 1, worin die Aralkylgruppe nachstehende Struktur hat: 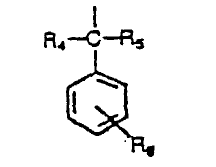 worin mindestens ein Rest aus R₄, R₅ und R₆ ausgewählt ist aus (a) Wasserstoff, (b) einer Alkylgruppe mit 1 bis 4 Kohlenstoffatomen und (c) Halogen.

3. Härtbares substituiertes Epoxyharz auf Resorcin-Basis nach Anspruch 1, enthaltend ein Härtemittel.

4. Verwendung eines härtbaren substituierten Epoxyharzes auf Resorcin-Basis nach Anspruch 3 als Klebemittel zum Kleben von Metall an Metall, Metall an Verbundwerkstoff oder Verbundwerkstoff an Verbundwerkstoff.

5. Verwendung eines härtbaren substituierten Epoxyharzes auf Resorcin-Basis nach Anspruch 3 in einer Beschichtungsformulierung.

6. Härtbares substituiertes Epoxyharz auf Resorcin-Basis nach Anspruch 3, worin das Härtemittel in einer Menge von 1 bis 15 Gew.-% vorliegt.

7. Härtbares substituiertes Epoxyharz auf Resorcin-Basis nach Anspruch 2, worin R₄ Methyl ist, R₅ entweder Wasserstoff oder Methyl und R₆ Wasserstoff.

8. Zähes beständiges Verbundwerkstoffteil, umfassend ein Matrixharz, Härtemittel und Füllmaterial, wobei das Matrixharz aus einem härtbaren substituierten Epoxyharz auf Resorcin-Basis nach Anspruch 1 besteht.

## Revendications

1. Résine époxy durcissable, à base de résorcinol substitué, ayant la formule développée : 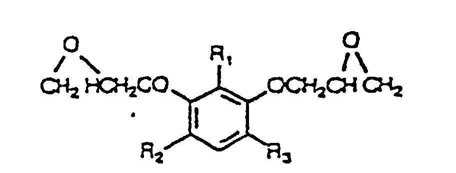 dans laquelle au moins l'un de R₁, R₂ et R₃ est choisi parmi (a) les groupes aralkyle, (b) le groupe benzoyle et (c) les groupes benzoyle substitués de structure : 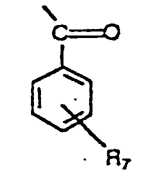 dans laquelle R₇ représente un atome d'halogène ou un groupe alkyle ayant 1 à 4 atomes de carbone.

2. Résine époxy à base de résorcinol substitué selon la revendication 1, pour laquelle le groupe aralkyle répond à la structure suivante : 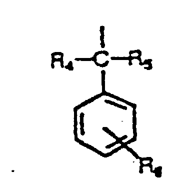 dans laquelle au moins l'un de R₄, R₅ et R₆ est choisi parmi (a) l'atome d'hydrogène, (b) les groupes alkyle ayant 1 à 4 atomes de carbone et (c) les atomes d'halogène.

3. Résine époxy durcissable, à base de résorcinol substitué, selon la revendication 1, à laquelle est incorporé un agent augmentant la résistance.

4. Utilisation d'une résine époxy durcissable, à base de résorcinol substitué, selon la revendication 3, en tant qu'adhésif pour lier du métal à du métal, du métal à un matériau composite ou un matériau composite à un autre matériau composite.

5. Utilisation d'une résine époxy durcissable, à base de résorcinol substitué, selon la revendication 3, dans une composition pour revêtement.

6. Résine époxy durcissable, à base de résorcinol substitué, selon la revendication 3, dans laquelle l'agent augmentant la résistance est présent à raison de 1 % à 15 % en poids.

7. Résine époxy durcissable, à base de résorcinol substitué, selon la revendication 2, dans laquelle R₄ représente un groupe méthyle, R₅ représente un atome d'hydrogène ou un groupe méthyle et R₆ représente un atome d'hydrogène.

8. Elément composite résistant, durable, qui comprend une matrice en résine, un agent augmentant la résistance et une charge, ladite matrice en résine étant constituée d'une résine époxy durcissable, à base de résorcinol substitué, telle que revendiquée dans la revendication 1.
